# EUROPEAN PATENT APPLICATION

(11) **EP 2 915 477 A1**
(43) Date of publication of application: **09.09.2015**
(21) Application number: 14849191.3
(22) Date of filing: 01.05.2014
(51) Int. Cl.: A61B 1/04, A61B 1/00, G02B 23/24

(54) **ELECTRICAL UNIT AND ENDOSCOPE HAVING SAME MOUNTED THERETO**

(30) Priority: 25.09.2013 JP 2013198388
(71) Applicant: OLYMPUS CORPORATION, Shibuya-ku Tokyo 151-0072 (JP)
(72) Inventor: TAKAHASHI, Tomohisa, Tokyo 151-0072 (JP)
(74) Representative: Schicker, Silvia
(86) International application number: PCT/JP2014/062051
(87) International publication number: WO 2015/045467

(57) **Abstract**

An electric unit according to the invention has a first and a second image pickup cables 46 and 47 having flexibility, a heat shrinkable tube 57 having a cable bundling portion 57a that bundles the first and the second image pickup cables 46 and 47, a first and a second substrates 44 and 45 to which the first and the second image pickup cables 46 and 47 are connected, an anti-mist element cable 58 and a heat radiation cable 60 that are bundled by the cable bundling portion 57a together with the first and the second image pickup cables 46 and 467 to protrude to sides of the first and the second substrates 44 and 45 from the cable bundling portion 57a, and a reinforcement frame 56 that fixes core wires 58a and 60a of both the cables 58 and 60, and both the cables 58 and 60 are placed in positions that are deviated from a center axis of the heat shrinkable tube 57 in the cable bundling portion 57a. Reference drawing: Fig. 5

## Description

### Technical Field

The present invention relates to an electric unit that makes a twist of a first cable, which occurs when the first cable is routed, difficult to transmit to an electric component side, and an endoscope loaded with the electric unit.

### Background Art

The functions of electronic equipment such as an endoscope are increasing more and more, and with the increase, the transmission signals to the electric unit represented by an image pickup apparatus or the like loaded on the electronic equipment increase, and the number of electric cables tends to increase inevitably.

When a relatively flexible long portion is included as in electronic equipment such as an endoscope, a plurality of electric cables are inserted through the long portion, and distal ends of the respective electric cables are connected to an electric unit that is placed at the distal end side of the electronic equipment, and when an operator routes the long portion of the electronic equipment, a twisting force in the rotational direction occurs to the respective electric cables which are placed inside, in the site where the long portion bends.

The twisting is transmitted to a distal end direction, but if there are a number of bending spots, the twisting force which is transmitted to the electric unit side becomes large correspondingly.

The twisting force is applied to the connection portion of the electric unit and the electric cable as a twist force, and in the electronic equipment showing such a behavior, the electric unit (a flexible printed board on which an electronic component is packaged, a solid image pickup device connecting to the flexible printed board, and the like), and the entire electric cable (image pickup cable) which connects to the electric unit are surrounded by an adhesive, and cured, whereby enhancement in durability is realized, as shown in International Publication No. 2010/064506, for example.

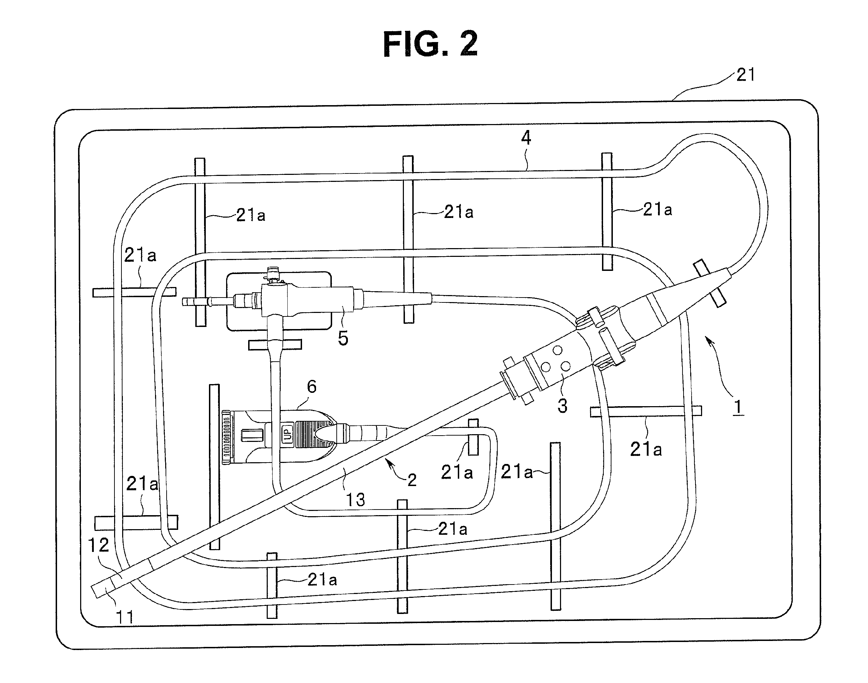

For example, a tray 21 (a detailed configuration will be described in embodiments) shown in Fig. 2 is a tray in which the endoscope 1 is set when autoclave sterilization (high pressure steam sterilization) treatment is performed for an endoscope 1 which is one example of the electronic equipment, and a universal cord 4 that is a long portion of the endoscope 1 is set in a state in which the universal cord 4 is routed in a predetermined manner.

An image pickup cable which performs transmission and reception of signals or the like with an image pickup apparatus which is provided at a distal end portion of an insertion portion 2, and the like are inserted through the inside of the universal cord 4 as an electric cable, and when two solid image pickup devices are placed in the image pickup apparatus, two of image pickup cables are placed to correspond to the two solid image pickup devices.

The image pickup cable has a large outside diameter, and is difficult to twist, and therefore, as the number of times of bending at the time of the universal cord 4 being set into the tray 21 increases, the twisting force which is transmitted to the distal end side of the insertion portion 2 gradually becomes large.

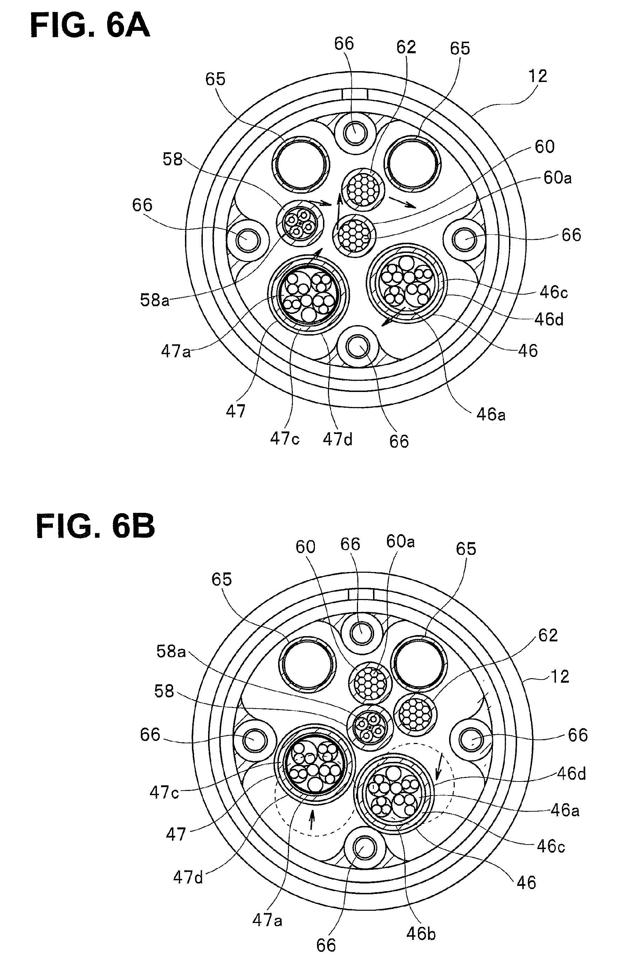

Incidentally, as shown in Fig. 6A and Fig. 6B, various cables 58, 60 and 62, a pair of light guide fibers 65, and four angle wires 66 that cause the bending portion 12 (see Fig. 2) which is provided at the distal end side of the insertion portion 2 to bend in an up, a down, a left and a right directions, and the like are inserted through the inside of the insertion portion 2 in a predetermined manner, besides the two image pickup cables 46 and 47.

Distal ends of the angle wires 66 are fixed to a distal end portion of the bending portion 12, but the other members are inserted through the insertion portion 2 in a state in which movement in a radial direction is relatively free.

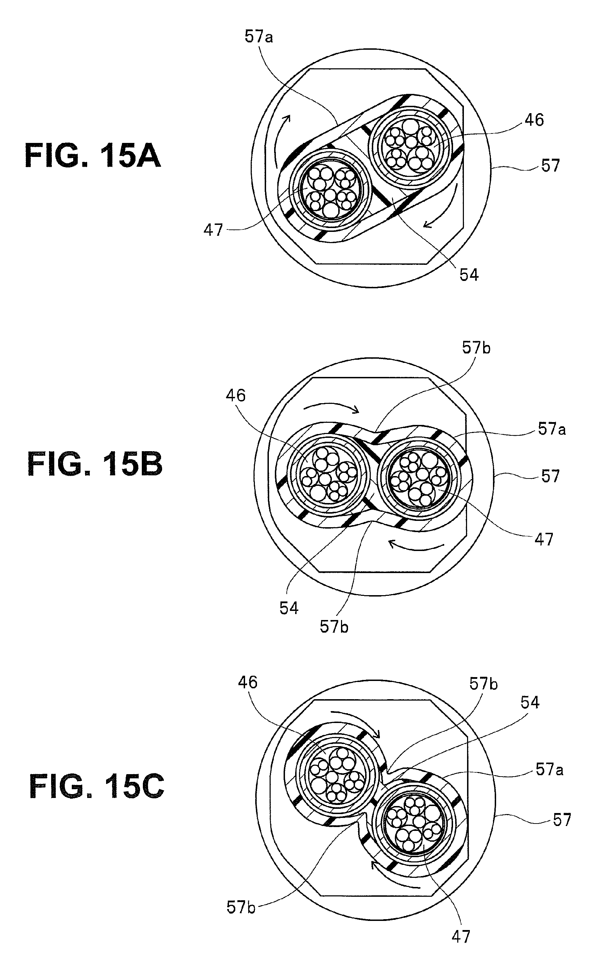

Among the members, the two image pickup cables 46 and 47 are bundled by a cable bundling portion 57a at a root side of a heat shrinkable tube 57 that covers the solid image pickup apparatus, and are hardened by an adhesive filler 54 which is filled in the heat shrinkable tube 57, at a side of the image pickup apparatus having the solid image pickup device, as shown in Fig. 15A to Fig. 15C.

As shown by the arrows in Fig. 15A, a twisting force from the universal cord 4 side is transmitted to both the image pickup cables 46 and 47 that are bundled by the cable bundling portion 57a.

The adhesive filler 54 has a peculiar softening point, and is easily softened when the endoscope 1 is heated at a temperature higher than the softening point as in autoclave treatment.

When the adhesive filler 54 is softened, both the image pickup cables 46 and 47 cannot resist the twisting force which is transmitted to the image pickup cables 46 and 47, and both the image pickup cables 46 and 47 start to move in the rotational direction together with the cable bundling portion 57a of the heat shrinkable tube 57, as shown in Fig. 15B.

Thereupon, deformation portions 57b in constricted shapes are formed in the cable bundling portion 57a.

When both the image pickup cables 46 and 47 further move in the rotational direction, a load is exerted on the cable bundling portion 57a, and the constrictions in the deformation portions 57b are gradually deformed by large amounts, as shown in Fig. 15C.

Further, with rotation of the image pickup cables 46 and 47, a twist force is easily applied to the connection portion of the image pickup cables 46 and 47 and the image pickup apparatus, and there is a possibility that breaking of a wire may occur, and a trouble such as core wires of the image pickup cables contacting each other and inducing a short circuit occurs.

Even when the adhesive filler is softened, the cable bundling portion of the heat shrinkable tube deforms, and a twist force occurs to the connection portion of the electric unit (the image pickup apparatus) and the electric cables (the image pickup cables), a trouble as described above does not occur immediately, but as a result that heating treatment is repeatedly applied to the electronic equipment (the endoscope), durability is reduced, and there arises the disadvantage of the product service life being shortened.

In the light of the above described circumstances, the present invention has an object to provide an electric unit, that are enhanced in durability, can realize long service life, and can obtain high reliability without an undue force being applied to a connection portion of the electric cable and the electric unit even when a twist force is transmitted to the electric unit side by routing of the electric cable, and an endoscope loaded with the electric unit.

### Disclosure of Invention

### Means for Solving the Problem

An electric unit according to one aspect of the present invention has first cables having flexibility, an outer sheath having a cable bundling portion that collectively surrounds and bundles a plurality of the first cables, an electric component to which a plurality of the first cables are connected, a twist preventing member that is bundled by the cable bundling portion together with the first cables, and has a rigid portion having a protruding portion that protrudes to a side of the electric component from the cable bundling portion, in at least one part, and a fixing section that fixes the protruding portion, wherein the rigid portion of the twist preventing member is placed in a position that is deviated from a center axis of the outer sheath in the cable bundling portion.

Further, an endoscope according to one aspect of the present invention is an endoscope loaded with the electric unit, wherein the electric component is an image pickup device provided at a distal end of an insertion portion and a substrate connected to the image pickup device.

### Brief Description of the Drawings

Fig. 1 is an entire configuration view of an endoscope according to a first embodiment;
Fig. 2 is a plan view of a state in which the endoscope is set in a heat treatment tray, according to the first embodiment;
Fig. 3 is a vertical sectional side view of a distal end portion of the endoscope, according to the first embodiment;
Fig. 4 is a horizontal sectional plan view of the distal end portion of the endoscope, according to the first embodiment;
Fig. 5 is a sectional view taken along line V-V in Fig. 4, according to the first embodiment;
Fig. 6A is a sectional view taken along line VI-VI in Fig. 1, and is a sectional view of a state before the endoscope is set into the heat treatment tray, according to the first embodiment;
Fig. 6B is a sectional view taken along line VI-VI in Fig. 1, and is a sectional view at a time of the endoscope being set into the heat treatment tray, according to the first embodiment;
Fig. 7 is a sectional view corresponding to Fig. 4, of a distal end portion of an endoscope according to a second embodiment;
Fig. 8 is a sectional view taken along line VIII-VIII in Fig. 7, according to the second embodiment;
Fig. 9 is a sectional view corresponding to Fig. 8, according to a third embodiment;
Fig. 10 is a sectional view corresponding to Fig. 8, according to a fourth embodiment;
Fig. 11 is a sectional view corresponding to Fig. 8, according to a fifth embodiment;
Fig. 12 is a sectional view corresponding to Fig. 8, according to a sixth embodiment;
Fig. 13 is a sectional view corresponding to Fig. 8, according to a seventh embodiment;
Fig. 14 is an essential part sectional side view of a distal end portion of an endoscope according to an eighth embodiment;
Fig. 15A is a sectional view of a cable bundling portion of a heat shrinkable tube that covers a conventional endoscope apparatus, and is an explanatory view of a twist force that acts on image pickup cables;
Fig. 15B is a sectional view of the cable bundling portion of the heat shrinkable tube which covers the conventional endoscope apparatus, and is an explanatory view of a state in which the image pickup cables rotate by softening of an adhesive filler; and
Fig. 15C is a sectional view of the cable bundling portion of the heat shrinkable tube which covers the conventional endoscope apparatus, and is an explanatory view of a state in which the cable bundling portion is deformed.

### Best Mode for Carrying Out the Invention

Hereinafter, one embodiment of the present invention will be described based on the drawings. Note that the drawings are schematic, attention should be paid to the fact that relations of thicknesses and widths of respective members, ratios of the thicknesses of the individual members and the like differ from actual relations and ratios, and among the drawings, parts in which the relations and ratios of mutual dimensions differ from one another are included as a matter of course.

### [First Embodiment]

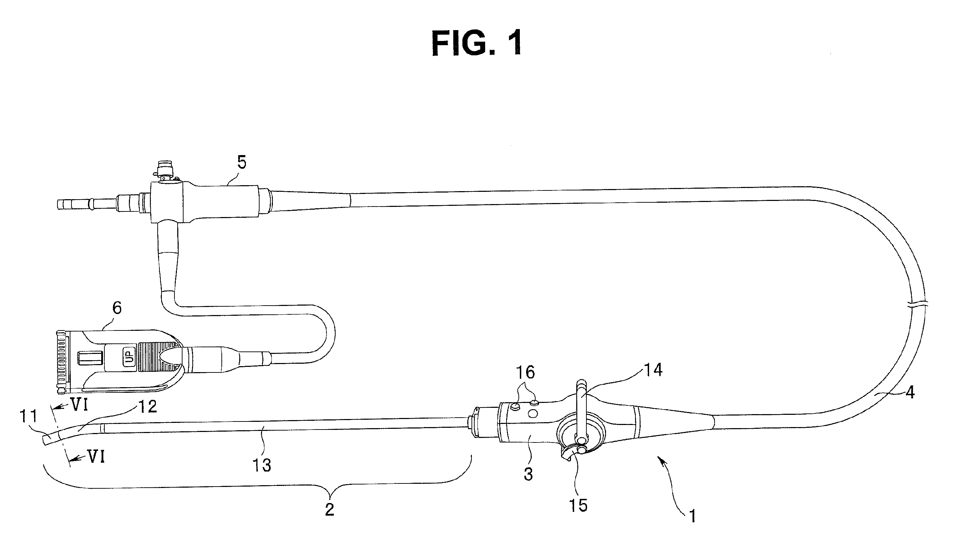

Fig. 1 to Fig. 6 show a first embodiment of the present invention.

Fig. 1 shows a rigid electronic endoscope (hereinafter, simply called "an endoscope") 1 capable of being subjected to autoclave (high pressure steam sterilization) treatment.

The endoscope 1 has an insertion portion 2, an operation portion 3 connectively provided at a proximal end of the insertion portion 2, a universal cord 4 that is extended from the operation portion 3, a scope connector 5 that is placed at a proximal end of the universal cord 4, and an electric connector 6 that is provided at an end portion of a cable extending from a side portion of the scope connector 5.

Further, the insertion portion 2 is configured by having a distal end portion 11, a bending portion 12 connectively provided at the distal end portion 11, and a rigid portion 13 provided between the bending portion 12 and the operation portion 3.

The operation portion 3 is provided with two bending operation levers 14 and 15 that perform bending operations of the bending portion 12 vertically and laterally by turning operations, and switches 16 for performing various operations and the like.

The switches 16 are operated when a predetermined endoscope function, for example, an operation or the like of an image pickup apparatus 31 that is placed in the distal end portion 11 is performed.

Further, when the endoscope 1 is accommodated in an autoclave apparatus (not illustrated) and autoclave treatment is applied to the endoscope 1, the endoscope 1 is set into a heat treatment tray 21 shown in Fig. 2.

In the heat treatment tray 21, a plurality of positioning portions 21a are provided as predetermined, which position the insertion portion 2 and the operation portion 3 as predetermined, and position the universal cord 4 and the like in a state in which the universal cord 4 and the like are routed as predetermined so that the insertion portion 2 and the operation portion 3 do not interfere with the long portion such as the universal cord 4.

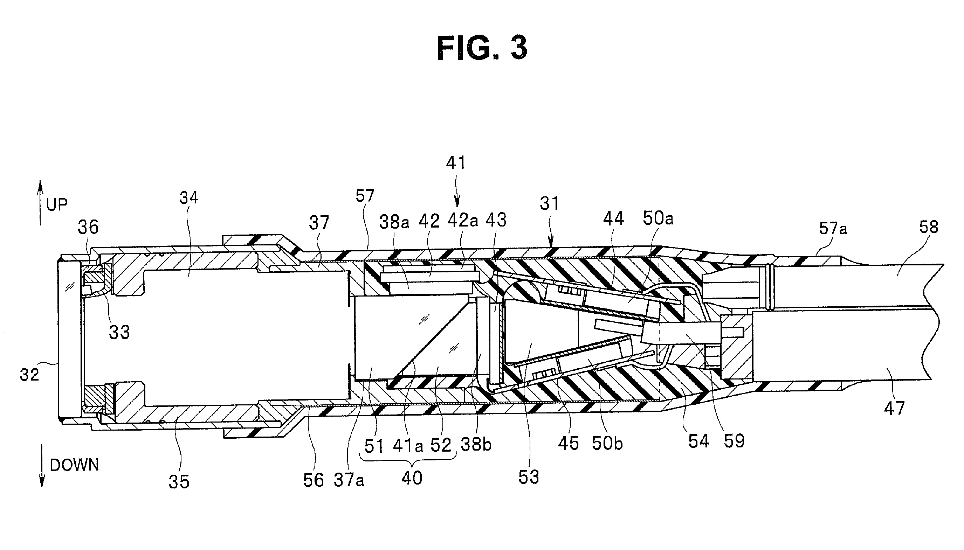

Further, as shown in Fig. 3, a distal end cover glass 32 that configures an observation window is provided at a distal end of the image pickup apparatus 31 which is incorporated in the distal end portion 11 of the endoscope 1, a ring-shaped anti-mist element 33 that heats the distal end cover glass 32 to perform demisting is placed at a rear portion of the distal end cover glass 32, and further, behind the anti-mist element 33, an objective lens unit 34 (details are not illustrated) including an objective lens, a retaining frame that retains an objective lens and the like is placed.

Further, an insulation frame 35 that retains the objective lens unit 34 is fitted and fixed to a metallic distal end frame 36 that retains the distal end cover glass 32 and the anti-mist element 33.

A distal end frame 36 is jointed to a proximal end outer circumferential portion of the insulation frame 35.

Further, an inner surface side of the insulation frame 35 is joined to a lens retaining frame (not illustrated), and an outer circumferential portion at a proximal end side of the lens retaining frame is jointed to a retaining holder 37.

Further, the image pickup apparatus 31 is placed at a proximal end portion of the retaining holder 37.

The image pickup apparatus 31 is a double image pickup apparatus including two solid image pickup devices, and has a prism unit 41 having a prism section 40, a first and a second solid image pickup devices 42 and 43 that are configured by image sensors such as CCD or CMOS, and a first and a second substrates 44 and 45 that are relatively rigid and a first and a second image pickup cables 46 and 47, and a distal end portion (a side on which an incident light from the objective lens unit 34 is incident) of the prism unit 41 is fitted and fixed to the proximal end portion of the retaining holder 37.

An electric component of the present invention is configured by the first and the second solid image pickup devices 42 and 43 and the first and the second substrates 44 and 45.

Further, a reinforcement plate 42a is bonded to a back surface of the first solid image pickup device 42.

The reinforcement plate 42a is for preventing breakage of the solid image pickup device 42 by a load at a time of assembly, and is formed from a relatively rigid insulating material such as glass and ceramics.

Note that in Fig. 3, the reinforcement plate 42a is shown as being in a flat plate shape, but the reinforcement plate 42a is not limited thereto, and the reinforcement plate 42a may be formed into a shape (a box shape, or a U-shape) covering the back surface and a side surface of the solid image pickup device 42.

The prism section 40 of the prism unit 41 is configured by joining a first prism 51 and a second prism 52 so as to emit an incident light transmitted through the objective lens unit 34 by dividing the incident light into two optical paths.

Further, the prism section 40 is configured by providing a green reflection coat layer (also called a dichroic coat layer) 41a on a joint boundary surface where the first prism 51 and the second prism 52 are layered on each other.

Note that the green reflection coat layer 41a is formed on the joint boundary surface where the first prism 51 and the second prism 52 are layered on each other by a reflection film being applied onto a slant surface of the first prism 51, and has a characteristic that reflects a light of green (G) of an incident light, and transmits lights of led (R) and blue (B).

The first solid image pickup device 42 for reproducing a luminance signal (a Y signal) is bonded and fixed to an exit surface at a side where substantially perpendicular reflection is performed by the green reflection coat layer 41 a of the first prism 51 via a first cover glass 38a, and the first solid image pickup device 42 receives light that is emitted from the first prism 51.

Further, the second solid image pickup device 43 for reproducing color signals (the R and B signals) is bonded and fixed to a rear part at a side (an exit surface side) where emission is performed after transmission through the green reflection coat layer 41 a of the first prism 51 and the second prism 52 via a second cover glass 38b, and receives light that is emitted by being transmitted through the first prism 51 and the second prism 52.

Note that though not illustrated, color filters for the red (R) and the blue (B) which are provided side by side in a stripe shape are provided on a light receiving surface of the second solid image pickup device 43, and thereby, the second solid image pickup device 43 functions as a solid image pickup device for reproducing color signals (the R and B signals).

Further, color filters are not provided on a light receiving surface of the first solid image pickup device 42, and accordingly, the first solid image pickup device 42 functions as a solid image pickup device for reproducing a luminance signal (a Y signal).

Further, as shown in Fig. 3, the first and the second substrates 44 and 45 on which electronic components 50a and 50b such as a capacitor, and an IC circuit are packaged are respectively connected to the first and the second solid image pickup devices 42 and 43.

The electronic components 50a and 50b are placed in a state in which the electronic components 50a and 50b confronting each other in a taper shape that opens to a distal end direction from a proximal end side, and a heat sink 53 is interposed between opposing surfaces thereof.

In the heat sink 53, surfaces that confront both the electronic components 50a and 50b are formed to be wedge-shaped in section which are parallel with the electronic components 50a and 50b, and a distal end face thereof is caused to confront a back surface of the second solid image pickup device 43, and promotes radiation of heat that is generated in the image pickup apparatus 31, and the electronic components 50a and 50b.

Further, a plurality of signal lines 46a which the first image pickup cable 46 has are electrically connected to the first substrate 44, and a plurality of signal lines 47a which the second image pickup cable 47 has are electrically connected to the second substrate 45.

The first image pickup cable 46 performs supply of electric power to the electronic component 50a and transmission and reception of signals to and from the first solid image pickup device 42 via the first substrate 44, and the second image pickup cable 47 performs supply of electric power to the electronic component 50b and transmission and reception of signals to and from the second solid image pickup device 43 via the substrate 45.

Note that as shown in Fig. 6A and Fig. 6B, the respective image pickup cables 46 and 47 are configured by the signal lines 46a and 47a as core wires with a plurality of element wires being twisted, internal insulating coatings 46b and 47b that cover the signal lines 46a and 47a, shielding wires 46c and 47c that cover the internal insulating coatings 46b and 47b, and external insulating coatings 46d and 47d that cover the shielding wires 46c and 47c.

Note that the first and the second image pickup cables 46 and 47 correspond to first cables having flexibility of the present invention.

Further, the prism section 40 is retained by a prism unit joining portion 37a of the retaining holder 37, and the prism unit joining portion 37a is provided with a reinforcement frame 56 of a metal having heat conductivity is provided to contain the respective substrates 44 and 45.

Note that the reinforcement frame 56 corresponds to an electric component retaining member as a fixing section of the present invention.

Further, an outer circumferential face of the retaining holder 37 is covered with a heat shrinkable tube 57 as an outer sheath.

The heat shrinkable tube 57 contains the reinforcement frame 56 and the image pickup apparatus 31, covers distal end outer circumferential portions where core wires are not exposed, of the various cables that are caused to face a proximal end portion side of the reinforcement frame 56, and bundles distal end portions of the respective cables.

The cables which are bundled by the heat shrinkable tube 57 include an anti-mist element cable 58 that supplies electric power to the anti-mist element 33, a heat radiation cable 60 and the like, besides the first and the second image pickup cables 46 and 47 described above.

Note that both the cables 58 and 60 correspond to a second cable of the present invention.

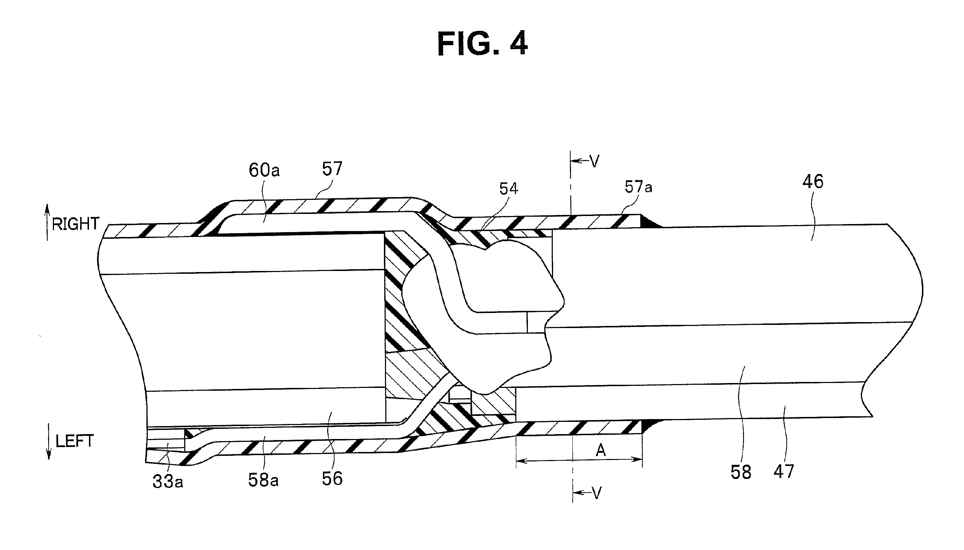

As shown in Fig. 4, a core wire 58a at a distal end side of the anti-mist element cable 58 is connected to a substrate 33a on which the anti-mist element 33 is packaged.

A core wire 60a at a distal end side of the heat radiation cable 60 is fixed to an outer periphery of the reinforcement frame 56 with use of solder, a heat conductive adhesive or the like.

The core wire 60a of the heat radiation cable 60 is formed with use of a material with favorable heat conductivity.

Further, as shown in Fig. 4, both the core wires 58a and 60a are exposed forward from an inside of a region A that is a range of a cable bundling portion 57a, are bent to intersect each other, and are fixed to a side surface of the reinforcement frame 56 with solder, an adhesive or the like.

Further, the core wires 58a and 60a are impregnated with solder to be made a rigid portion having protruding portions.

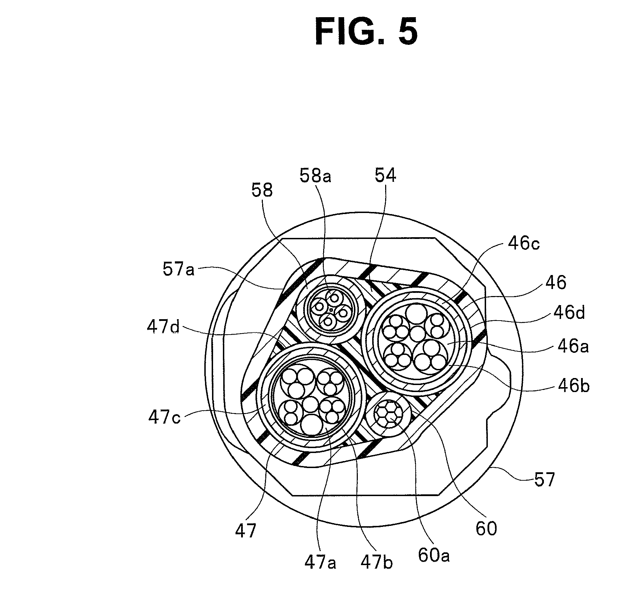

Further, as shown in Fig. 5, in the cable bundling portion 57a, the first and the second image pickup cables 46 and 47 are placed adjacently to each other, and the anti-mist element cable 58 and the heat radiation cable 60 are placed in an opposed state at both sides with a line connecting centers of both the image pickup cables 46 and 47 therebetween.

Both the cables 58 and 60 are fixed by being collectively surrounded and bundled by the cable bundling portion 57a together with the image pickup cables 46 and 47 in a state in which the respective image pickup cables 46 and 47 contact both the cables 58 and 60.

As above, in the present embodiment, the core wires 58a and 60a of the anti-mist element cable 58 and the heat radiation cable 60 are made the rigid portion having the protruding portions by being impregnated with solder and are fixed to the reinforcement frame 56, and the proximal portion sides thereof are bundled in the cable bundling portion 57a and fixed in the state in which the proximal portion sides are caused to confront each other with the image pickup cables 46 and 47 therebetween, whereby the anti-mist element cable 58 and the heat radiation cable 60 are caused to function as a twist preventing member.

Further, since the existing anti-mist element cable 58 and the heat radiation cable 60 are caused to function as the twist preventing member, reduction in the number of components is achieved, and reduction of a diameter of the insertion portion 2 can be further realized.

Further, the proximal end sides of the first and the second image pickup cables 46 and 47, and the anti-mist element cable 58 are connected to the electric connector 6 via the insertion portion 2, the operation portion 3, the universal cord 4 and the scope connector 5.

The proximal end side of the heat radiation cable 60 undergoes insulation treatment, and is disposed in the scope rigid portion 13.

Heat that is transmitted to the reinforcement frame 56 is further transmitted to the heat radiation cable 60.

The anti-mist element cable 58 supplies heating electric power from a power supply (not illustrated) that is connected to the electric connector 6 to the anti-mist element unit 33.

An adhesive filler 54 is filled in the reinforcement frame 56, and by the adhesive filler 54, the respective components configuring the image pickup apparatus 31 are sealed in the reinforcement frame 56, and is integrally fixed firmly.

A material of the adhesive filler 54 is an epoxy resin, for example, having an insulation property.

Further, the heat sink 53 and a shielding wire 46c of the first image pickup cable 46 are electrically connected via a heat radiating jumper wire 59.

Further, a shielding wire 47c of the second image pickup cable 47 is also electrically connected to the heat sink 53 via another heat radiating jumper wire.

Thereby, part of heat of the heat sink 53 is also released to outside by the shielding wires 46c and 47c.

Next, an operation of the present embodiment which is composed of the configuration as above will be described.

When the endoscope 1 is set into the heat treatment tray 21 in order that heat treatment such as autoclave treatment is applied to the endoscope 1, the universal cord 4 is routed in a predetermined manner, and is engaged with respective positioning portions 21 a, as shown in Fig. 2.

Thereupon, the first and the second image pickup cables 46 and 47, the anti-mist element cable 58, the heat radiation cable 60, another cable 62 and the like which are internally fitted into the universal cord 4 and the insertion portion 2 are inserted through the universal cord 4 and the insertion portion 2 in a state in which movement in a radial direction is relatively free, and therefore, twisting occurs each time the cables are bent.

In this case, the anti-mist element cable 58 and the heat radiation cable 60 have relatively small outside diameters and are flexible in addition, and therefore, stress that occurs in a rotational direction is absorbed by twisting of themselves.

However, the first and the second image pickup cables 46 and 47 have large outside diameters, and difficult to twist, and therefore, as the number of bending times at a time of routing the universal cord 4 increases, a twisting force which is transmitted to the distal end side gradually becomes large.

Incidentally, as shown in Fig. 6A and Fig. 6B, the first and the second image pickup cables 46 and 47, the anti-mist element cable 58 and the heat radiation cable 60 which are described above, the other cable 62, a pair of light guide fibers 65, four angle wires 66 that cause the bending portion 12 to bend a vertical and a lateral directions, and the like are inserted through the inside of the insertion portion 2 in a predetermined manner.

Distal ends of the angle wires 66 are fixed to the distal end portion 12a of the bending portion 12, but the other members are inserted in a state in which movement in the radial direction is relatively free.

Therefore, in a vicinity of the cable bundling portion 57a of the heat shrinkable tube 57, a rotational force as shown by the arrows in Fig. 6A occurs to the respective image pickup cables 46 and 47 by the twisting force which is transmitted from the rear end side, whereby as shown in Fig. 6B, the anti-mist element cable 58, the heat radiation cable 60 and the other cable 62 which are incorporated therein are pushed away and are moved, and the pair of light guide fibers 65 are easily crushed because the pair of light guide fibers 65 are relatively flexible.

In this case, the image pickup apparatus 31 which is placed in the distal end portion 11 is fixed into the reinforcement frame 56 by the adhesive filler 54, and the distal end outer circumferential portions where the core wires of the respective cables 46, 47, 58 and 60 are not exposed are collectively surrounded, bundled and fixed by the cable bundling portion 57a of the heat shrinkable tube 57, and therefore, in the vicinity of the cable bundling portion 57a, both the image pickup cables 46 and 47 do not move significantly to the rotational direction as shown in Fig. 6B.

However, when heat treatment is applied to the endoscope 1 which is set into the heat treatment tray 21 in the predetermined manner, at a temperature higher than a softening point of the adhesive filler 54 which seals the image pickup apparatus 31, by sterilizing treatment represented by autoclave treatment or the like, the adhesive filler 54 is softened as a matter of course, whereby internal stress (a twist force) accumulated in the respective image pickup cables 46 and 47 cannot resist and are gradually released, and in the cable bundling portion 57a of the heat shrinkable tube 57, both the image pickup cables 46 and 47 are to rotate in a state in which both the image pickup cables 46 and 47 are fastened by the cable bundling portion 57a as in Fig. 15 (b) described above.

Subsequently, the twist force (the rotational force) is transmitted to the connection portion with the image pickup apparatus 31, a twist (twisting) shearing force easily occurs, and durability is gradually reduced by repetition of the heat treatment.

In relation to the above, in the present embodiment, as shown in Fig. 4 and Fig. 5, the core wires 58a and 60a of the anti-mist element cable 58 and the heat radiation cable 60 are exposed forward from the inside of the region A which is the range of the cable bundling portion 57a and are fixed to the side surfaces of the reinforcement frame 56 with solder, an adhesive or the like, and the core wires 58a and 60a are further impregnated with solder to be made the rigid portion.

Therefore, in the cable bundling portion 57a, the anti-mist element cable 58 and the heat radiation cable 60 function as the twist preventing member, and even when the first image pickup cable 46 and the second image pickup cable 47 are to rotate, the rotation is inhibited.

As a result, the cable bundling portion 57a is not deformed, a shearing force does not occur to the connection portion with the image pickup apparatus 31, and even when heat treatment is repeated, durability is not significantly reduced.

That is to say, for example, when both the image pickup cables 46 and 47 are to rotate in a clockwise direction in Fig. 5, the first image pickup cable 46 contacts the anti-mist element cable 58, and the rotation thereof is inhibited.

The second image pickup cable 47 contacts the heat radiation cable 60, and the rotation thereof is inhibited.

When both the image pickup cables 46 and 47 are to rotate in a counterclockwise direction, the second image pickup cable 47 contacts the anti-mist element cable 58 and is inhibited, and the first image pickup cable 46 contacts the heat radiation cable 60 and is inhibited.

As a result, the existing anti-mist element cable 58 and the existing heat radiation cable 60 can inhibit the twist rotational forces in both directions of both the image pickup cables 46 and 47, and durability of the entire endoscope 1 can be enhanced.

### [Second Embodiment]

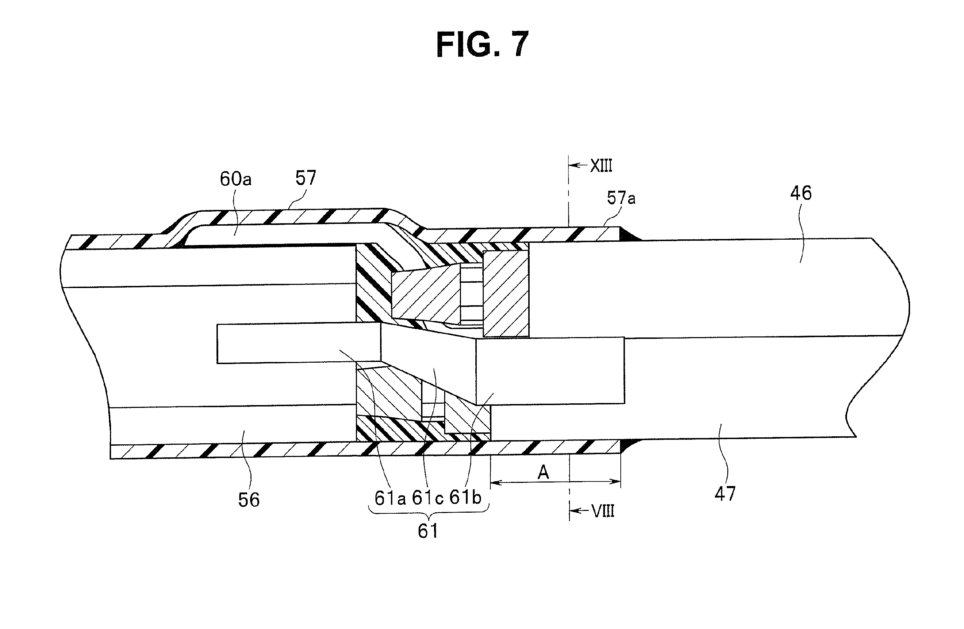

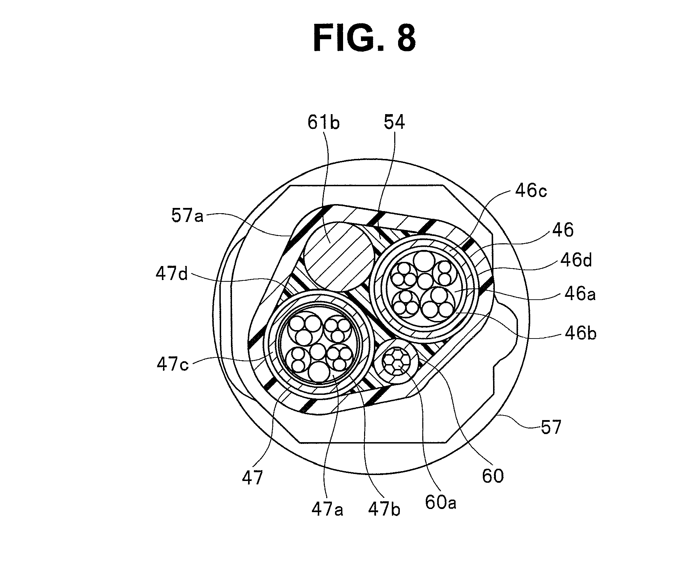

Fig. 7 and Fig. 8 show a second embodiment of the present invention.

In the aforementioned first embodiment, the existing anti-mist element cable 58 and the existing heat radiation cable 60 are caused to function as the twist preventing member, but in the present embodiment, an exclusive twist preventing member 61 is placed in place of the anti-mist element cable 58, and the twist preventing member 61 and the heat radiation cable 60 as the second cable are configured to inhibit rotational movement of the two image pickup cables 46 and 47.

Depending on the endoscope 1 to be used, the aforementioned anti-mist element cable 58 is not needed.

In the case as above, the cable bundling portion 57a of the heat shrinkable tube 57 fastens and bundles the first image pickup cable 46 and the second image pickup cable 47, and the heat radiation cable 60, and at this time, an effect similar to the effect of the aforementioned first embodiment can be obtained by use of the exclusive twist preventing member 61 in substitution for the aforementioned anti-mist element cable 58.

That is to say, as shown in Fig. 7 and Fig. 8, as for the twist preventing member 61, a distal end portion 61a of the twist preventing member 61 as a protruding portion is fixed to the outer periphery of the reinforcement frame 56 with use of solder, an adhesive or the like.

The twist preventing member 61 prevents the twist forces which are transmitted from the respective image pickup cables 46 and 47 from being transmitted to the image pickup apparatus 31 side, and is formed from a rigid material.

The twist preventing member 61 is formed into a rod shape, a rear end portion 61b is protruded rearward from the reinforcement frame 56, and an end portion thereof is caused to face a same spot as a rear end of the cable bundling portion 57a formed at a tube rear end portion of the heat shrinkable tube 57, or a slightly distal end side from the rear end of the cable bundling portion 57a, that is, an inside of the region A which is the range of the cable bundling portion 57a.

A bent portion 61 c is formed in a middle thereof, and by the bent portion 61 c, a position in the radial direction which the rear end portion 61b is caused to face is set.

As shown in Fig. 8, in the cable bundling portion 57a, the heat radiation cable 60 is placed at one of positions that are facing each other with a line connecting axes of both the image pickup cables 46 and 47 therebetween, and the rear end portion 61 b of the twist preventing member 61 is caused to face the heat radiation cable 60 in the other position.

The heat radiation cable 60 and the rear end portion 61b of the twist preventing member 61 are brought into contact with the respective image pickup cables 46 and 47, and are fixed in the state in which the rear end portion 61 b and the respective image pickup cables 46 and 47 are surrounded and bundled collectively.

The distal end portion 61 a of the twist preventing member 61 is fixed to the reinforcement frame 56 with use of solder, an adhesive agent or the like.

Note that in the twist preventing member 61 according to the present embodiment, a diameter of the rear end portion 61b becomes thicker as compared with the distal end portion 61a, and the diameters of the rear end portion 61b and the distal end portion 61 a are properly set in accordance with sectional diameters of gaps which the rear end portion 61 b and the distal end portion 61 a are caused to face.

In the configuration as above, the twist preventing member 61 is applied in place of the anti-mist element cable 58 of the first embodiment, and therefore, even with the endoscope 1 which does not include the anti-mist element cable 58, an effect equivalent to the effect of the first embodiment can be obtained.

Note that in the present embodiment, the twist preventing member 61 is applied in place of the anti-mist element cable 58, but in an endoscope that does not include the heat radiation cable 60, the twist preventing member 61 is used in substitution for the heat radiation cable 60.

### [Third Embodiment]

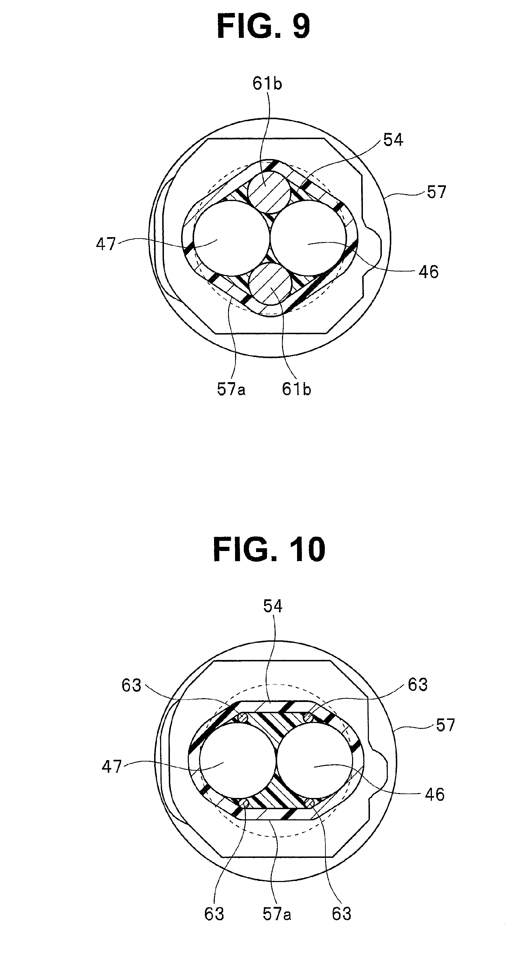

Fig. 9 shows a third embodiment of the present invention.

Depending on the endoscope 1 to be used, both the anti-mist element cable 58 and the heat radiation cable 60 which are described above are not needed.

In the case as above, an effect similar to the effect of the aforementioned first embodiment can be obtained with use of a pair of exclusive twist preventing members 61 in substitution for both the cables 58 and 60.

Further, the rear end portion 61b of the twist preventing member 61 according to the present embodiment is smaller than the diameters of both the image pickup cables 46 and 47, and accordingly a center of the rear end portion 61b is placed at a position that deviates from a center axis of the heat shrinkable tube 57 in the cable bundling portion 57a.

As above, in the present embodiment, the exclusive twist preventing members 61 are provided, whereby even with the endoscope in which both the anti-mist element cable 58 and the heat radiation cable 60 are not placed, an effect similar to the effect of the first embodiment can be obtained.

### [Fourth Embodiment]

Fig. 10 shows a fourth embodiment of the present invention.

In the aforementioned third embodiment, the rear end portions 61 b of the two twist preventing members 61 are disposed to be in contact with the respective image pickup cables 46 and 47, but in the present embodiment, four twist preventing members 63 formed from a rigid material are placed with respect to the two image pickup cables 46 and 47.

Note that though not illustrated, distal end portions of the respective twist preventing members 63 are fixed to the reinforcement frame 56 (see Fig. 7).

That is to say, as shown in Fig. 10, the twist preventing members 63 are respectively caused to face each other in spaces between the outer peripheries of the respective image pickup cables 46 and 47 and the cable bundling portion 57a of the heat shrinkable tube 57 in a direction substantially perpendicular to a line connecting centers of both the image pickup cables 46 and 47.

Thereby, even when the respective image pickup cables 46 and 47 are to rotate in either direction, the rotation is inhibited here by the respective twist preventing members 63.

In the present embodiment, the number of twist preventing members 63 increases as compared with the aforementioned third embodiment, but the rotational force is dispersed to the individual twist preventing members 63, and therefore, the diameters can be made small correspondingly.

### [Fifth Embodiment]

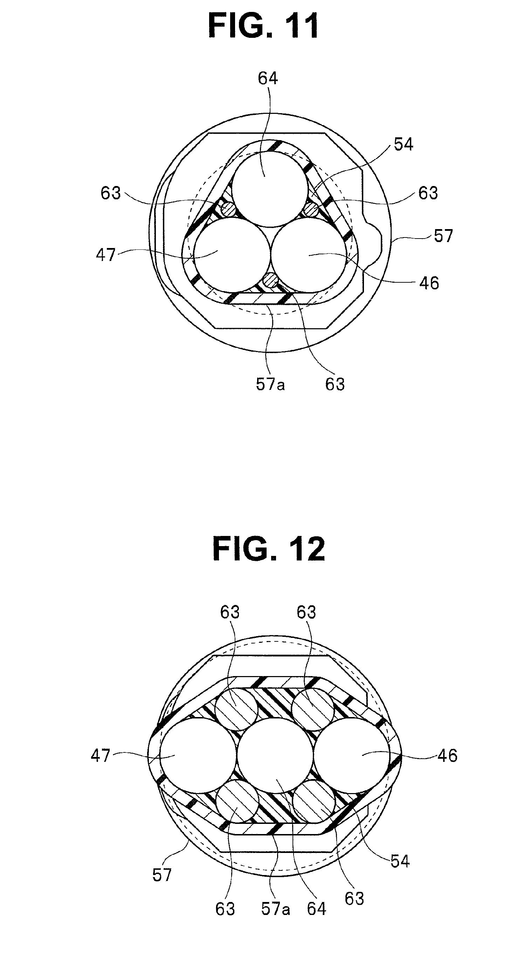

Fig. 11 shows a fifth embodiment of the present invention.

In the first to the fourth embodiments described above, the aspect in which the two image pickup cables 46 and 47 are bundled and fastened by the cable bundling portion 57a of the heat shrinkable tube 57 is described, but in the present embodiment, an aspect in which a third image pickup cable 64 as the first cable having flexibility is bundled by the cable bundling portion 57a in addition to the two image pickup cables 46 and 47 is shown.

That is to say, the respective image pickup cables 46, 47 and 64 are bundled in a triangular shape in a state in which the respective image pickup cables 46, 47 and 64 are in contact with one another, the twist preventing members 63 are placed at outer circumferential sides where the adjacent image pickup cables 46, 47 and 64 are in contact with one another in a state in which the twist preventing members 63 are in contact with the two adjacent image pickup cables 46, 47 and 64.

Note that a broken line shows a region in the radial direction in which the respective image pickup cables 46, 47 and 64 are to rotate.

According to the present embodiment, even when the respective image pickup cables 46, 47 and 64 are to rotate in either direction, the rotational movement thereof can be reliably inhibited by the respective twist preventing members 63, similarly to the aforementioned fourth embodiment.

### [Sixth Embodiment]

Fig. 12 shows a sixth embodiment of the present invention.

In the aforementioned fifth embodiment, the aspect in which the three image pickup cables 46, 47 and 64 are bundled in the triangular shape is described, but in the present embodiment, the three image pickup cables 46, 47 and 64 are bundled in a laterally arranged state.

That is to say, in the present embodiment, each pair of twist preventing members 63 are placed to face each other at outer circumferential sides of a site where the respective image pickup cables 46, 47 and 64 which are adjacent to one another with the respective image pickup cables 46, 47 and 64 therebetween, the respective twist preventing members 63 are brought into contact with the image pickup cables 46, 47 and 64 which are adjacent to one another, and are bundled and fastened collectively by the cable bundling portion 57a.

According to the present embodiment, movement of the respective image pickup cables 46, 47 and 64 is restricted by the respective twist preventing members 63, and therefore, even if the adhesive filler 54 is softened at the time of heat treatment, a single-line state of the three image pickup cables 46, 47 and 64 can be kept.

As a result, the image pickup cables 46, 47 and 64 are easily fastened and fixed in the single-line state, and the respective image pickup cables 46, 47 and 64 do not move even if heat treatment is repeatedly performed, whereby favorable durability can be obtained.

### [Seventh Embodiment]

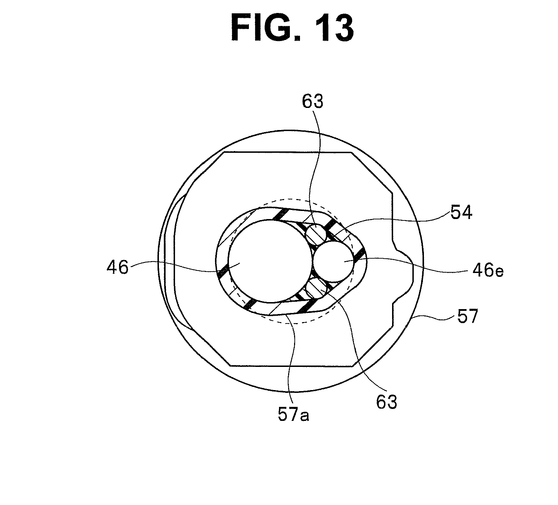

Fig. 13 shows a seventh embodiment of the present invention.

In the aforementioned third embodiment, the aspect in which the two image pickup cables 46 and 47 have the same diameter is described, but the present embodiment shows an aspect in which two image pickup cables 46 and 46e having different diameters are bundled.

When the two image pickup cables 46 and 46e having different diameters are bundled, the image pickup cable 46e having a small diameter moves more easily in the rotational direction as compared with the image pickup cable 46 having a large diameter, and therefore, the diameter of the twist preventing member 63 is set to be such a diameter that the image pickup cable 46e does not ride over the twist preventing member 63 when a rotational force occurs to the image pickup cable 46e having a small diameter.

The other operation is the same as the operation of the aforementioned second embodiment, and therefore, explanation will be omitted.

### [Eighth Embodiment]

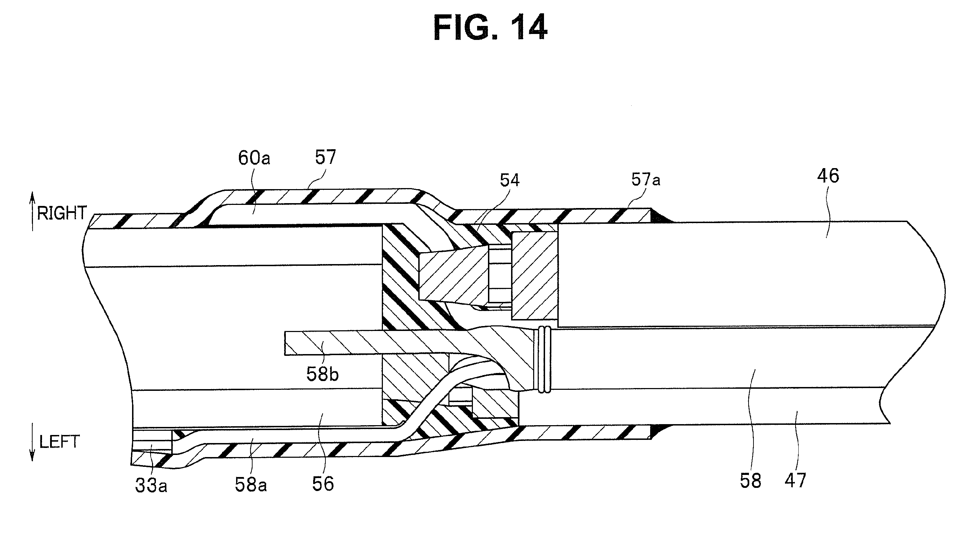

Fig. 14 shows an eighth embodiment of the present invention.

The present embodiment is a modification of the aforementioned seventh embodiment.

The present embodiment is such that a shielding wire 58b which is coated with an internal insulating film not illustrated and is provided at the anti-mist element cable 58 is caused to protrude, is collectively made a stranded wire, is impregnated with solder to be made a rigid portion having a protruding portion, and is fixed to the reinforcement frame 56 with use of solder or an adhesive agent.

Thereby, the shielding wire 58b of the anti-mist element cable 58 can be also caused to function as the twist preventing member.

As a result, rotational movement of the two image pickup cables 46 and 47 can be inhibited more reliably by the existing anti-mist element cable 58.

Note that the present invention is not limited to the aforementioned respective embodiments, and, for example, the fixing section may be a site other than the reinforcement frame 56 if only the fixing section is a relatively firm site.

The present application claims the benefit of Japanese Patent Application No. 2013-198388 filed in Japan on September 25, 2013, the entire contents of which are incorporated in the description, claims, and the drawings of the present application by reference.

## Claims

1. An electric unit, comprising:
first cables having flexibility;
an outer sheath having a cable bundling portion that collectively surrounds and bundles a plurality of the first cables;
an electric component to which a plurality of the first cables are connected;
a twist preventing member that is bundled by the cable bundling portion together with the first cables, and has a rigid portion having a protruding portion that protrudes to a side of the electric component from the cable bundling portion, in at least one part; and
a fixing section that fixes the protruding portion,
wherein the rigid portion of the twist preventing member is placed in a position that is deviated from a center axis of the outer sheath in the cable bundling portion.

2. The electric unit according to claim 1,
wherein the fixing section is the electric component.

3. The electric unit according to claim 1,
wherein the fixing section is an electric component retaining member that directly or indirectly retains the electric component.

4. The electric unit according to claim 1,
wherein the twist preventing member is at least one second cable that is bundled by the outer sheath, and
the rigid portion is a portion formed by an element wire of the second cable being solidified with solder.

5. An endoscope loaded with the electric unit according to any one of claims 1 to 4,
wherein the electric component is an image pickup device provided at a distal end of an insertion portion and a substrate connected to the image pickup device.
